# EUROPEAN PATENT SPECIFICATION

(11) **EP 0 484 832 B1**
(45) Date of publication and mention of the grant of the patent: **11.03.1998**
(21) Application number: 91118671.6
(22) Date of filing: 31.10.1991
(51) Int. Cl.: C08K 5/3492, C07D 251/18

(54) **Self-extinguishing polymeric compositions**
Selbstverlöschende polymere Zusammensetzungen
Compositions polymères auto-extinctrices

(30) Priority: 02.11.1990 IT 2196690
(43) Date of publication of application: 13.05.1992
(73) Proprietor: MINISTERO DELL' UNIVERSITA' E DELLA RICERCA SCIENTIFICA E TECNOLOGICA, 00196 Roma (IT)
(72) Inventor: Cipolli, Roberto, Dr., I-28100 Novara (IT); Masarati, Enrico, Dr., I-29010 Castelnuovo Valtidone, Piacenza (IT); Nucida, Gilberto, I-20098 San Giuliano Milanese, Milan (IT); Oriani, Roberto, I-20137 Milan (IT); Pirozzi, Mario, Dr., I-20097 San Donato Milanese, Milan (IT)
(74) Representative: Barz, Peter, Dr.

(56) References cited:
- EP-A- 0 053 775
- EP-A- 0 326 082
- EP-A- 0 441 135
- US-A- 3 206 407
- US-A- 3 301 797

## Description

The present invention relates to self-extinguishing polymeric compositions based on thermoplastic polymers and/or polymers endowed with elastomeric properties, especially olefinic polymers and copolymers, which compositions comprise specific triazine derivatives in combination with ammonium and/or amine phosphates and/or ammonium and/or amine phosphonates.

Several methods of reducing or eliminating the combustibility of polymers are known in the art. Some of said methods are based on the use of metal compounds, in particular on antimony, bismuth or arsenic, in combination with partially halogenated, thermally unstable organic compounds, such as chlorinated paraffin waxes.

Other methods are based on the use of substances capable of yielding intumescence. The formulations of the intumescent type generally are composed of the polymer and at least three main additives, i.e., an essentially phosphorus-containing additive, whose purpose is to form, during the combustion, an impermeable, semi-solid and vitreous layer essentially composed of polyphosphoric acid and to activate the process of formation of intumescence; a second additive, containing nitrogen, which is to serve as foaming agent; and a third, carbon-containing additive, which acts as a carbon donor in order to allow an insulating cellular carbonaceous layer ("char") to be formed between the polymer and the flame.

Examples of intumescent formulations of said type may be found in US-A-3,810,862 (Phillips Petroleum Co.; based on melamine, pentaerythritol and ammonium polyphosphate); US-A-4,727,102 (Vamp S.r.l.; based on melamine cyanurate, a hydroxyalkyl derivate of isocyanuric acid and ammonium polyphosphate); and WO-85/05626 (Plascoat U.K. Limited; on the basis of various phosphorus and nitrogen compounds among which, in particular, a combination of melamine phosphate, pentaerythritol and ammonium polyphosphate may be mentioned).

In more recent formulations, together with an organic or inorganic phosphorus compound, a nitrogen-containing organic compound was used, generally consisting of an aminoplastic resin obtained by means of condensation of urea, melamine or dicyandiamide with formaldehyde.

Examples of formulations containing two additives are those described in US-A-4,504,610 (Montedison S.p.A.; based on oligomeric derivatives of 1,3,5-triazine and ammonium polyphosphate) and EP-A-14,463 (Montedison S.p.A.; based on organic compounds selected from benzylguanamine and reaction products of aldehydes and several cyclic nitrogen compounds, in particular benzylguanamine-formaldehyde copolymers, and ammonium polyphosphate).

Self-extinguishing compositions can also be obtained by using single-component additives, which contain in their organic molecule both nitrogen and phosphorus atoms, as disclosed in US-A-4,201,705 (Borg-Warner Corp.).

These intumescent flame retardant systems endow the polymers to which they are added with the property of forming a carbonaceous residue when they burn or are exposed to a flame. This kind of flame-retardant system offers numerous advantages, i.e., absence of corrosion phenomena in the machinery in which the polymers are processed, a lower emission of smokes as compared to systems containing metal compounds and halogenated hydrocarbons, and, above all, the possibility of endowing the polymers with satisfactory flame-proof properties with a smaller amount of total additive and therefore without excessively impairing the mechanical properties thereof.

It has now surprisingly been found that polymeric compositions having very good anti-flame properties can be obtained by using certain oligomeric compounds derived from 2,4-diamino-6-hydroxy-1,3,5-triazine, the effectiveness of said oligomers being higher than that of prior art products.

Accordingly, the present invention provides compositions comprising:
a) from 89 to 40 (preferably 83 to 50) parts by weight of thermoplastic polymer and/or of polymer having elastomeric properties;
b) from 8 to 33 (preferably 12 to 30) parts by weight of one or more ammonium and/or amine phosphates and/or phosphonates;
c) from 3 to 27 (preferably 5 to 20) parts by weight of one or more oligomeric compounds having 2 to 50 (and preferably 10 to 30) repeating units of general formula (I): 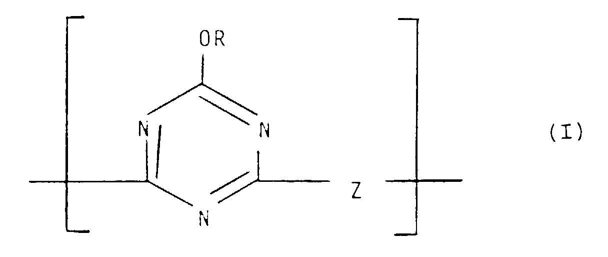
wherein:
R = is hydrogen; (C₂-C₆)-alkenyl; (C₆-C₁₂)-cycloalkyl; (C₆-C₁₂)-alkylcycloalkyl; (C₆-C₁₂)-aryl; (C₇-C₁₂)-aralkyl; or ⁅CₘH₂ₘ⁆Y, m being an integer of from 1 to 8, preferably from 1 to 4, and
Y being selected from hydrogen; CN; -O(C₁-C₄)-alkyl; -O(C₂-C₄)-alkenyl; (C₆-C₁₂)-cycloalkyl; (C₆-C₁₂)-alkylcycloalkyl; -O(C₆-C₁₂)-aryl; -N(R₁)₂, wherein the groups R₁, equal or different from each other, are (C₁-C₄)-alkyl or (C₃-C₄)-alkenyl; and an N-heterocyclic radical linked to the alkyl chain through the nitrogen atom and optionally containing another heteroatom, preferably selected from O, S and N; and
Z is a divalent or polyvalent radical selected from those of the following formulae: 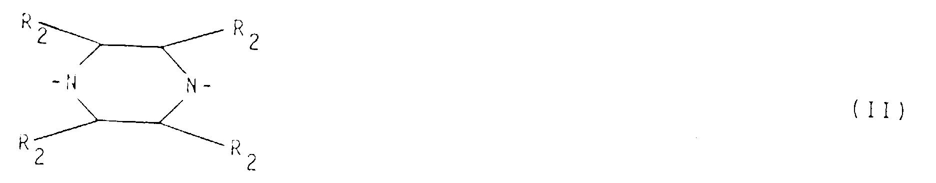
wherein the groups R₂, the same or different from each other, represent hydrogen and (C₁-C₄)-alkyl; 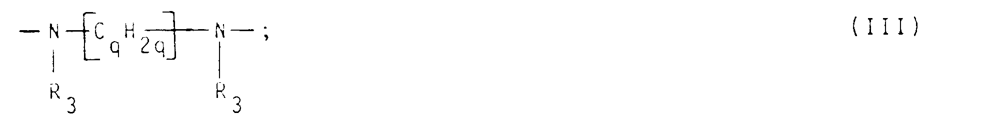 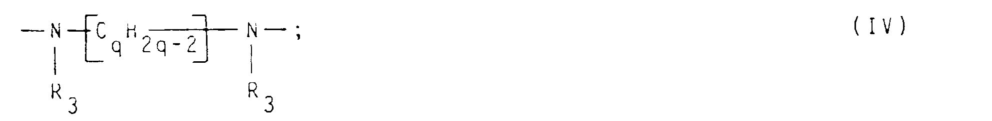 wherein q is an integer of from 2 to 14 and R₃ is hydrogen; (C₁-C₄)-alkyl; (C₂-C₆)-alkenyl; or (C₁-C₄)-hydroxyalkyl; 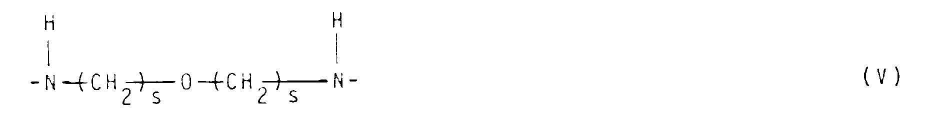 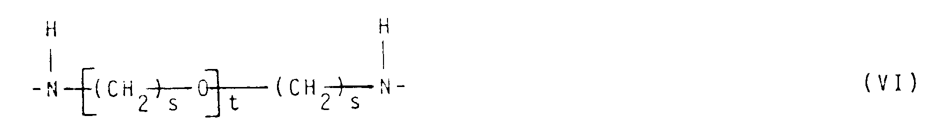 wherein s is an integer of from 2 to 5 and t is an integer of from 1 to 3; 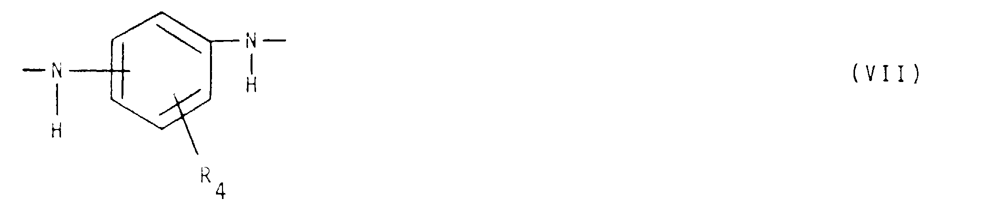 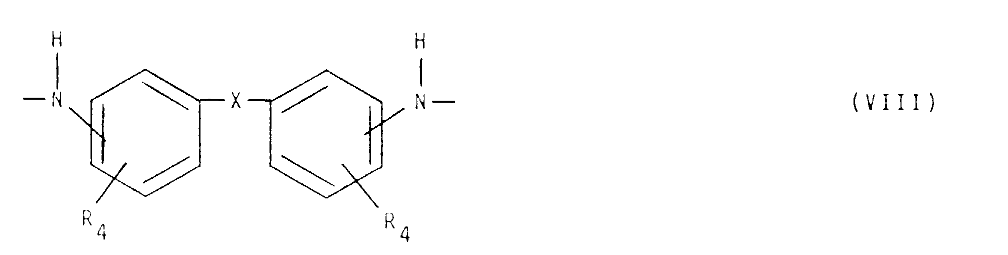 wherein:
X is a direct carbon-carbon bond (C-C); O; S; S-S; SO; SO₂; NH; NHSO₂; NHCO; N=N; or CH₂; and
R₄ is hydrogen; hydroxy; (C₁-C₄)-alkyl; or (C₁-C₄)-alkoxy; 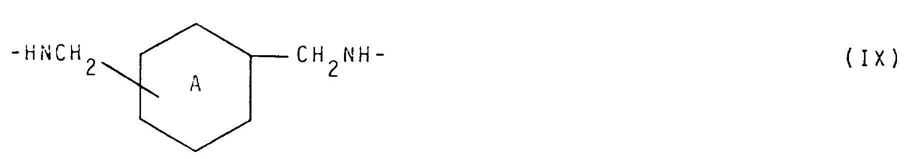
wherein A is a saturated or unsaturated (e.g. aromatic) ring; 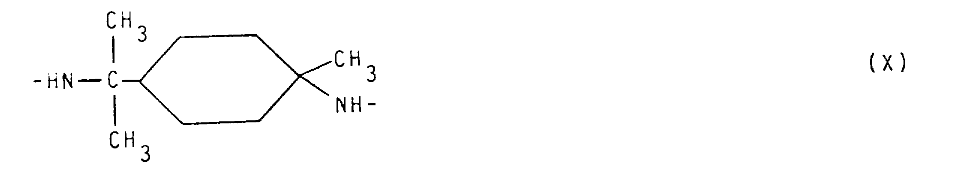 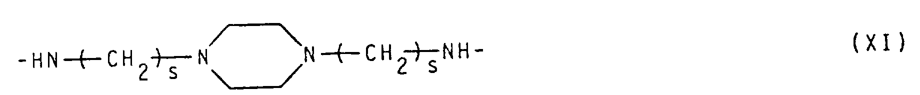 wherein s has the previously defined meaning; 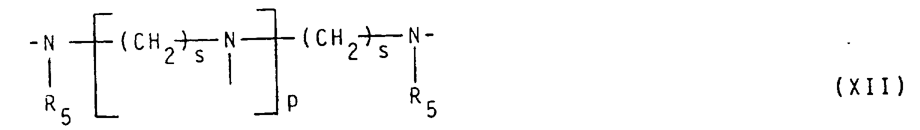 wherein R₅ is hydrogen or (C₁-C₄)-alkyl; p is an integer of from 1 to 5; and the subscripts s, the same or different from each other, have the previously defined meaning; 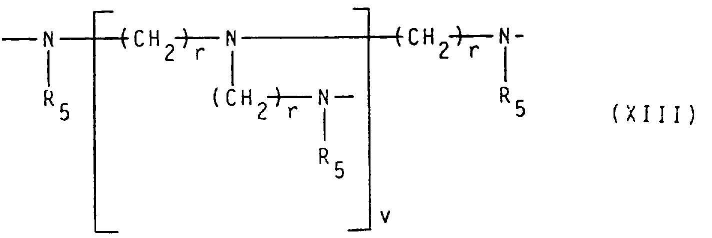 wherein :
- R₅: has the previously defined meaning;
- r: is an integer of from 2 to 4; and
- v: is 1 or 2.

The above oligomers having repeating units of general formula (I) are particularly stable to heat and therefore retain a high activity as flame retardants even after the heat processing of polymeric compositions containing them.

Furthermore, the compositions of the present invention show the advantage of emitting, in the case of a fire, only a very moderate amount of (non-darkening) smokes.

Specific examples of the group R in general formula (I) are methyl; ethyl; propyl; isopropyl; n-butyl; isobutyl; tert-butyl; n-pentyl; isopentyl; n-hexyl; tert-hexyl; octyl; tert-octyl; ethenyl; propenyl; butenyl; isobutenyl; hexenyl; cyclohexyl; propylcyclohexyl; butylcyclohexyl; phenyl; benzyl; 2-phenyl-ethyl; cyanomethyl; 2-cyanoethyl; 2-methoxyethyl; 2-methoxypropyl; 3-methoxypropyl; 4-methoxybutyl; 5-methoxypentyl; 6-methoxyhexyl; 7-methoxyheptyl; 7-methoxyoctyl; 2-ethoxyethyl; 3-ethoxypropyl; 4-ethoxybutyl; 5-ethoxypentyl; 3-propoxypropyl; 3-butoxypropyl; 4-butoxybutyl; 4-isobutoxybutyl; 2-propoxpentyl; 2-cyclohexyloxyethyl; 2-ethenyloxyethyl; 2-phenoxyethyl; 2-(N,N-dimethylamino)ethyl; 3-(N,N-dimethylamino)propyl; 4-(N,N-dimethylamino)butyl; 5-(N,N-dimethylamino)pentyl; 6-(N,N-dimethylamino)hexyl; 2-(N,N-diethylamino)ethyl; 3-(N,N-diethylamino)propyl; 4-(N,N-diethylamino)butyl; 5-(N,N-diethylamino)pentyl; 5-(N,N-diisopropylamino)pentyl; 4-(N,N-dipropylaminobutyl); 2-(N,N-diisopropylamino)ethyl; 2-(N,N-dipropylamino)ethyl; 2-(N-methyl-N-1-propenylamino)ethyl; 2-(N,N-di-1-propenylamino)ethyl; 4-(N,N-di-1-propenylamino)butyl; etc.

Specific examples of N-heterocyclic radicals Y are pyrrolidyl; piperidyl; morpholyl; thiomorpholyl; piperazyl; 4-methylpiperazyl; 4-ethylpiperazyl; etc.

Specific examples of groups -Z- are those deriving from the removal of a hydrogen atom from each reacted amino group of the following compounds: piperazine; 2-methylpiperazine; 2,5-dimethylpiperazine; 2,3,5,6-tetramethylpiperazine; 2-ethylpiperazine; 2,5-diethylpiperazine; 1,2-diaminoethane; 1,3-diaminopropane; 1,4-diaminobutane; 1,5-diaminopentane; 1,6-diaminohexane; 1,8-diaminooctane; 1,10-diaminodecane; 1,12-diaminododecane; N,N'-dimethyl-1,2-diaminoethane; N-methyl-1,3-diaminopropane; N-ethyl-1,2-diaminoethane; N-isopropyl-1,2-diaminoethane; N-(2-hydroxyethyl)-1,2-diaminoethane; N,N'-bis(2-hydroxyethyl)-1,2-diaminoethane; N-(2-hydroxyethyl)-1,3-diaminopropane; N-hexenyl-1,6-diaminohexane; N,N'-diethyl-1,4-diamino-2-butene; 2, 5-diamino-3-hexene; 2-aminoethylether; (2-aminoethoxy)methylether; 1,2-bis(2-aminoethoxy)ethane; 1, 3-diaminobenzene; 1,4-diaminobenzene; 2,4-diaminotoluene; 2,4-diaminoanisole; 2,4-diaminophenol; 4-aminophenylether; 4,4'-methylenedianiline; 4,4'-diaminobenzanilide; 3-aminophenylsulfone; 4-aminophenylsulfone; 4-aminophenylsulfoxide; 4-aminophenyldisulfide; 1,3-bis(aminomethyl)benzene; 1,4-bis(aminomethyl)benzene; 1,3-bis(aminomethyl)cyclohexane; 1,8-diamino-p-menthane; 1,4-bis(2-aminoethyl)piperazine; 1,4-bis(3-aminopropyl)piperazine; 1,4-bis(4-aminobutyl)piperazine; 1,4-bis(5-aminopentyl)piperazine; bis(2-aminoethyl)amine; bis(3-aminopropyl)amine; bis(4-aminobutyl)amine; bis(5-aminopentyl)amine; bis[2-(N-methylamino)ethyl]amine; 2-N-butyl-bis(2-aminoethyl)amine; bis[3-(N-methylamino)propyl]amine; N-(3-aminopropyl)-1,4-diaminobutane; N-(3-aminopropyl) -1,5-diaminopentane; N-(4-aminobutyl)-1,5-diaminopentane; tris(2-aminoethyl)amine; tris(3-aminopropyl)amine; tris(4-aminobutyl)amine; tris[2-(N-ethylamino)ethyl]amine; N,N'-bis(2-aminoethyl)-1,2-diaminoethane; N,N'-bis(3-aminopropyl)-1,3-diaminopropane: N,N'-bis(2-aminoethyl)-1,3-diaminopropane, N,N'-bis(3-aminopropyl)-1,2-diaminoethane;N,N'-bis(3-aminopropyl)-1,4-diaminobutane; bis[2-(2-aminoethyl)aminoethyl]amine; N,N'-bis[2-(2-aminoethyl)aminoethyl]-1,2-diaminoethane; N,N'-bis[3-(2-aminoethyl)aminopropyl]-1,2-diaminoethane; N,N,N',N'-tetrakis(2-aminoethyl)-1,2-diaminoethane; etc.

Particularly preferred are units of general formula (I) wherein R₁ is hydrogen or ⁅CₘH₂ₘ⁆Y, m being an integer cf from 1 to 4 and Y being hydrogen.

Oligomeric compounds having n repeating units covered by general formula (I) which are not specified in the examples, but are equally advantageously useable in the self-extinguishing polymeric compositions of the present invention are those listed in Table 1 below. In said table n is the number of repeating units of general formula (I).

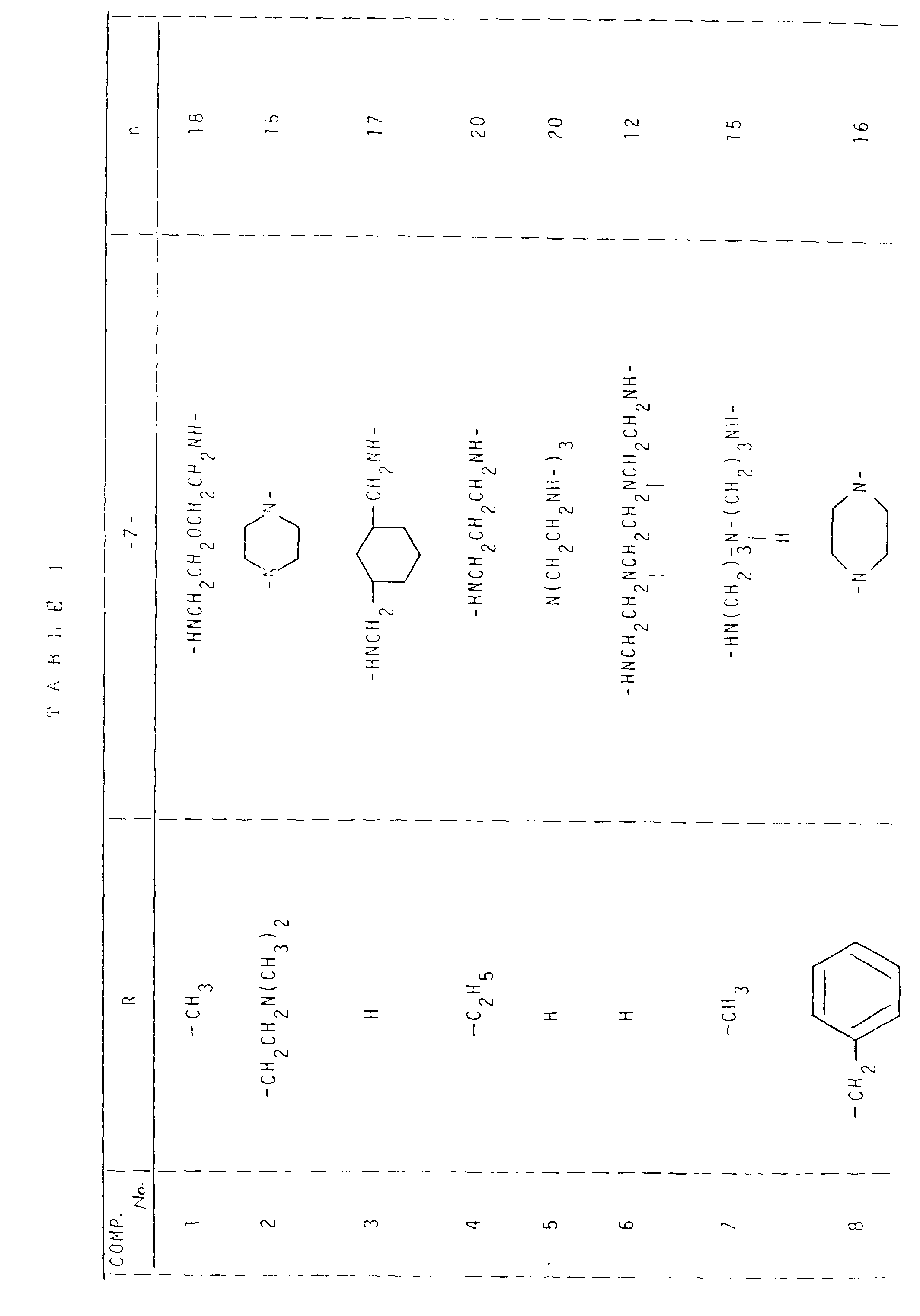

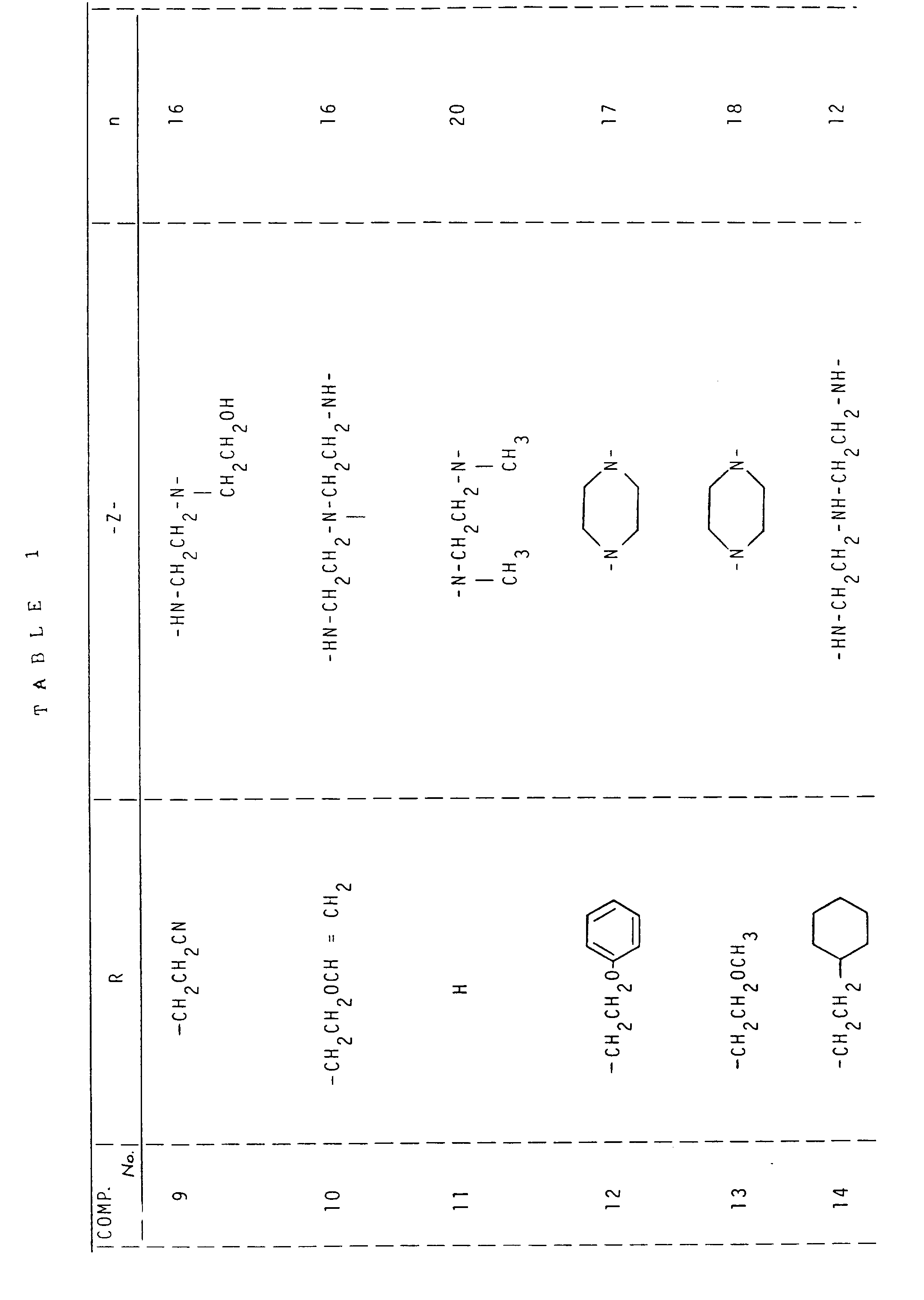

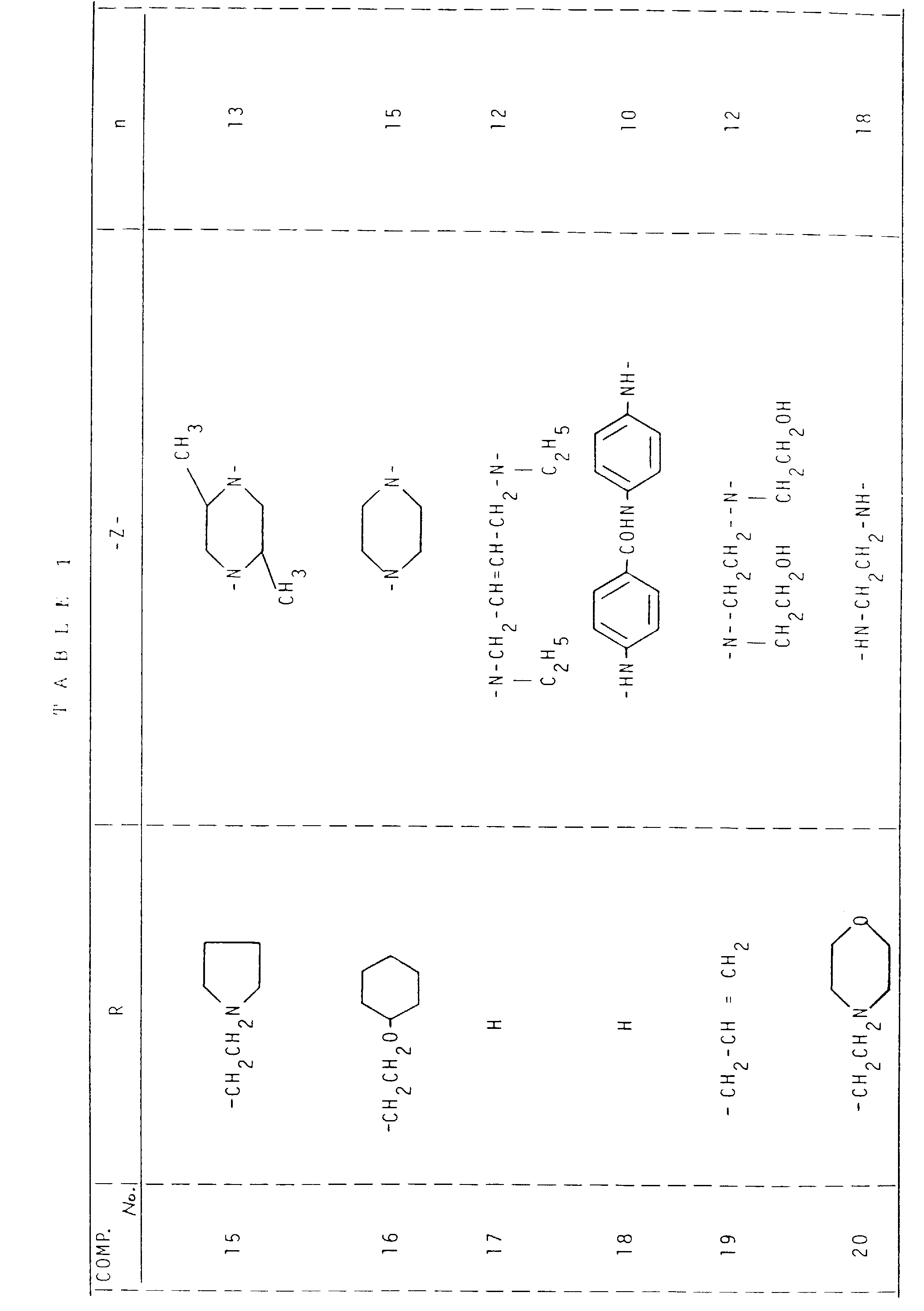

When R is different from hydrogen the compounds with repeating units of general formula (I) can be prepared by reacting a cyanuric acid halide, for example the chloride, with a compound of general formula (XIV):

R―OH (XIV)

wherein R has the previously defined meaning, at temperatures of from about 10 to about 110°C in a suitable solvent (such as acetone, methylene chloride, toluene, xylene, etc.) or in an excess of the compound (XIV) if the latter can act as solvent (as in the case of, e.g., methanol, ethanol, etc.) in the presence of an acidity acceptor (such as NaOH, NaHCO₃, Na₂CO₃, triethylamine, collidine, etc.), thereby obtaining the intermediate of general formula (XV): 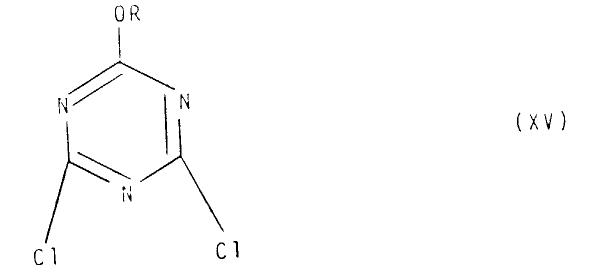 This intermediate, either separated or not, is allowed to react, under conditions analogous to the preceding ones but working at temperatures of from about 0 to about 150°C and with solvents which are compatible with said temperatures (such as acetonitrile, water, xylene, ortho-dichlorobenzene, etc.), with a polyamine of general formula (XVI):

H―Z―H (XVI)

wherein Z is as defined above, in the presence of an acidity acceptor.

The molar ratio of intermediate (XV) to polyamine (XVI) generally ranges from about 1:1 (for linear oligomers) up to a maximum of about 3.5:1 (for cross-linked oligomers).

An alternative method comprises the reaction of a cyanuric acid halide, for instance the chloride, with a polyamine of general formula (XVI) as defined above, in a suitable molar ratio and always under conditions analogous to those described previously, at temperatures of from about 0 to about 60°C, to afford the intermediate of general formula (XVII): 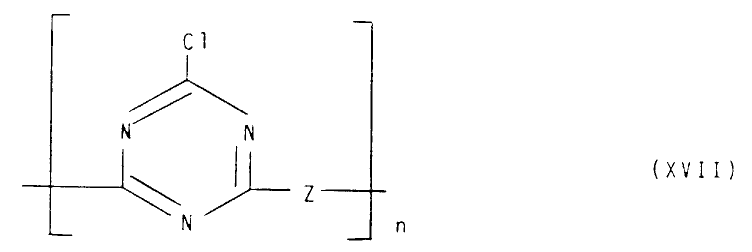 wherein Z and n are as defined above.

From this intermediate, either separated or not, the compounds of general formula (I) may be obtained according to the following procedure:
a) when R is hydrogen, by hydrolysis reaction either with an acid (such as hydrochloric acid, hydrobromic acid, sulfuric acid, phosphoric acid, etc.) at temperatures of from about 60 to about 100°C, or with a base (such as sodium hydroxide, potassium hydroxide, etc.) at temperatures of from about 100 to about 180°C;
b) when R is different from hydrogen, by condensation reaction with a compound of general formula (XIV) as defined above, in a suitable solvent (such as toluene, xylene, ortho-dichlorobenzene, etc.) or in an excess of the compound (XIV) if the latter can act as solvent (such as in the case of methanol, ethanol, etc.) and in the presence of a base (such as sodium hydroxide, potassium hydroxide, sodium metal, etc.) at temperatures of from about 60 to about 150°C.

Starting from the oligomers with repeating units of general formula (I) wherein R is different from hydrogen and preferably is (C₁-C₄)-alkyl, it is possible to obtain the corresponding oligomers wherein R is hydrogen by hydrolysis reaction, either with an acid, working at temperatures of from about 80 to about 140°C, or with a base, working at temperatures of from about 100 to about 180°C, using the reagents already indicated for the hydrolysis of the intermediates of general formula (XVII).

Among the phosphates, ammonium polyphosphates of the general formula

(NH₄)ₙ₊₂PₙO₃ₙ₊₁

wherein n is an integer equal to, or greater than, 2, are preferred. The molecular weight of the polyphosphates should be high enough to secure a low solubility in water.

For exemplary purposes, n preferably is comprised within the range of from 2 to 500.

The composition of the polyphosphates having the formula indicated hereinabove, in which n is a high enough number, and preferably ranges from 5 to 500, practically corresponds to a metaphosphate of formula

(NH₄PO₃)ₙ.

An example of said polyphosphates is the product known under the trade name "Exolit ® 422" (Hoechst), having the composition (NH₄PO₃)ₙ in which n is greater than 50. Another example is the product known under the name "Phos-Chek ® P/30" (Monsanto Chemical) which has a similar composition.

Another polyphosphate which can be used advantageously, above all thanks to its low solubility in water, is the product known under the trade name "Exolit ® 462" (Hoechst) which is "Exolit ® 422" microencapsulated in melamine-formaldehyde resin.

Other phosphates which can be used are those which are derived from amines, such as, e.g., dimethyl ammonium phosphate or diethyl ammonium phosphate, ethylene diamine phosphate, melamine orthophosphate or melamine pyrophosphate.

Good results may also be obtained by using mono- or polyammonium phosphonates selected from the salts derived from mono- or polyphosphonic acids.

Examples of said acids are:
ethane-1,1,2-triphosphonic acid, 2-hydroxy-ethane-1,1,2-triphosphonic acid, propane-1,2,3-triphosphonic acid, methylphosphonic acid, ethylphosphonic acid, n-propylphosphonic acid, n-butylphosphonic acid, phenylphosphonic acid, 1-amino-ethane-1,1-diphosphonic acid, 1-hydroxy-ethane-1,1-diphosphonic acid, 1-hydroxy-dodecane-1,1-diphosphonic acid, phosphonoacetic acid, 2-phosphonopropionic acid, 3-phosphonopropionic acid, 2-phosphonobutyric acid, 4-phosphonobutyric acid, amine tri(methylenephosphonic) acid, ethylenediamine tetra(methylenephosphonic) acid, hexamethylene diamine tetra(methylenephosphonic) acid, diethylene triamine penta(methylenephosphonic) acid, etc.

Among the polymers which can be used in the compositions according to the present invention, preferred are polymers and copolymers of olefins of general formula

R' - CH = CH₂

wherein R' is hydrogen or a (C₁₋₈)-alkyl or (C₆₋₁₀)-aryl radical, in particular:
(1) isotactic or prevailingly isotactic polypropylene;
(2) HDPE, LLDPE and LDPE polyethylene;
(3) crystalline copolymers of propylene and minor amounts of ethylene and/or of other alpha-olefins, such as, e.g., 1-butene, 1-hexene, 1-octene, 4-methyl-1-pentene;
(4) heterophasic compositions comprising (A) a fraction constituted by a propylene or ethylene homopolymer, or by one of the copolymers defined under (3); and (B) a copolymeric fraction constituted by elastomeric copolymers of ethylene and an alpha-olefin, optionally containing minor proportions of one or more dienes, wherein the alpha-olefin is preferably selected from propylene and 1-butene; and
(5) elastomeric copolymers of ethylene and alpha-olefin(s), optionally containing minor proportions of one or more dienes.

Examples of dienes more commonly contained in said elastomeric copolymers are butadiene, ethylidene-norbornene and hexadiene-1,4.

Among the polymers of olefins of general formula R'-CH=CH₂ wherein R' is an aryl radical, "crystal" and impact-resistant polystyrene are preferred.

Other examples of polymers which can be used are acrylonitrile/butadiene/styrene terpolymers (ABS) ; styrene/acrylonitrile copolymers (SAN) ; polyurethanes (of polyester or polyether grade) ; poly(ethylene terephthalate); poly(butylene terephthalate); polyamides; etc.

The self-extinguishing compositions according to the present invention can be prepared according to methods known in the art. For example, ammonium and/or amine phosphate and/or phosphonate is first intimately mixed with one or more finely ground nitrogen-containing compounds having units of general formula (I) (the particles of which generally are smaller than 70 µm) and the thus obtained mixture is added to the polymer in a turbo-mixer, in order to generate a homogeneous mixture which is either extruded or granulated. The thus obtained granular product can be transformed into various finished articles according to any of the well-known moulding techniques.

The fire-retardant additives according to the present invention are also suitable for use in the field of flame-retardant paints.

The following non-limitative examples are to further illustrate the present invention.

### EXAMPLE 1

Into a 2 l reactor equipped with stirrer, thermometer, feeding funnel, condenser and cooling bath, 600 ml of methanol, 80 ml of water and 100.8 g of sodium bicarbonate are introduced.

The mixture is cooled to 10°C and 110.7 g of cyanuric acid chloride are added. The temperature is allowed to rise up to 30°C and is kept at this value for about 1 hour, until the evolution of carbon dioxide is complete.

The exothermy of the reaction is sufficient to maintain the desired temperature.

The resulting reaction mixture is cooled to 5°C and subsequently 700 ml of cold water are added.

The product formed is filtered off and washed on the filter with cold water.

By drying the cake in an oven under vacuum at 60°C, 92.1 g of the intermediate of formula (XVIII): 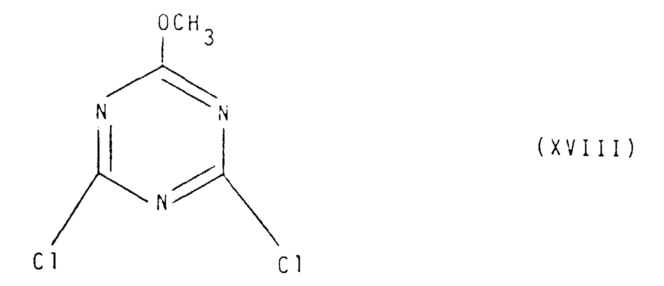 are obtained in the form of a white crystalline powder having a melting point (m.p.) of 90-92°C and a chlorine content of 39.27% (theoretical: 39.44%).

Furthermore, the structure of the intermediate (XVIII) was confirmed by NMR analysis.

In the above 2 l reactor, this time equipped with heating bath, 400 ml of acetonitrile and 72.0 g of the intermediate (XVIII) are introduced.

The solution is cooled to 5°C and a solution of 24.0 g of ethylene diamine in 100 ml of acetonitrile is added over about 1 hour.

Thereafter the temperature is raised to 70°C and a solution of 84.8 g of sodium carbonate in 250 ml of water is added over 3 hours whereafter the mixture is heated to boiling and is kept under reflux for about 3 hours.

Then the distillation of acetonitrile is started, keeping the volume of the mixture unchanged by adding water. The whole mass is then cooled to 50°C and the product formed is filtered and washed on the filter with water at 50°C.

By drying the cake in an oven at 100°C, 66.2 g of the product of formula 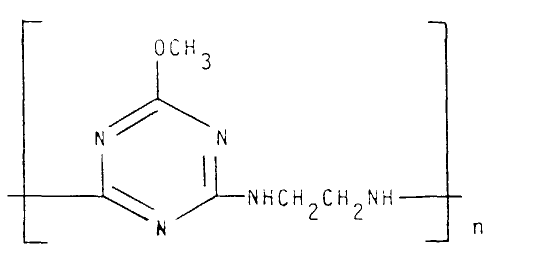 wherein n = 25, are obtained in the form of a white crystalline powder having a m.p. higher than 300°C.

### EXAMPLE 2

Into a 0.5 l reactor equipped as described in example 1, 200 ml of water, 78.8 g of a 37% by weight hydrochloric acid and 66.8 g of the product obtained in example 1 are introduced.

The mass is heated to boiling and is maintained under reflux for about 16 hours.

At the end the mass is cooled to 50°C and is neutralized by adding a solution of 32 g of sodium hydroxide in 100 ml of water. The resulting mixture is kept under stirring for a further 30 minutes, whereafter the product formed is filtered and washed on the filter with water at 50°C.

By drying the cake in an oven at 100°C, 56.9 g of the product of formula 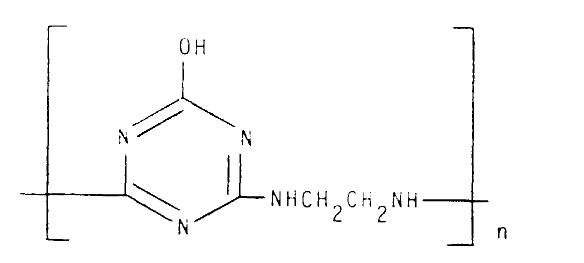 wherein n = 25, are obtained in the form of a white crystalline powder having a m.p. higher than 300°C.

### EXAMPLE 3

In a 2 l reactor equipped like that of example 1 there are introduced 800 ml of xylene, 50 ml of water and 90.9 g of the intermediate (XVIII) followed, under stirring, by the addition of 43.0 of piperazine over about 20 minutes.

The temperature of the dispersion rises up to 35-40°C; by means of the external bath the dispersion is kept at 40°C and is maintained under agitation for about 1 hour.

Thereafter, 20.0 g of sodium hydroxide dissolved in 40 ml of water are added within 2 hours and at 40°C.
Subseqeuntly the temperature is raised to 80°C and the mixture is maintained under stirring at this temperature for about 1 hour.

Further 20.0 g of sodium hydroxide dissolved in 40 ml of water are then added over 2 hours, whereafter the temperature is gradually raised up to 120 - 125°C while removing the water azeotropically.

The temperature is kept at this value for about 2 hours, then the whole mass is cooled to room temperature and the product formed is filtered.

The filter cake is thoroughly squeezed and then is thoroughly washed with water.
After drying in an oven at 100°C, 91.8 g of the product of formula 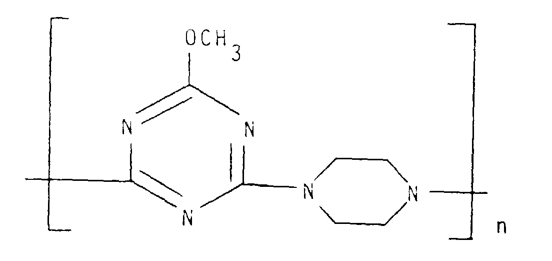 wherein n = 20, are obtained in the form of a white crystalline powder having a m.p. higher than 300°C.

### EXAMPLE 4

In a 1 l reactor equipped like that of example 1, 400 ml of acetone, 92.2 g of cyanuric acid chloride and 53.3 g of n-butanol are introduced.

The mixture is cooled to 0°C and within about 1 hour 60.5 g of collidine are added thereto while keeping the temperature within the range of from 0 to 5°C.

The whole mass is kept under agitation at 5°C for a further hour and then the temperature is allowed to rise up to room temperature.

After agitation for a further two hours, the collidine hydrochloride formed is removed by filtration.
The acetone solution is then poured into 500 ml of cold water and treated with 3 portions of 200 ml each of ethyl ether.

The ether extracts are collected and then the distillation thereof is carried out by separating at first the ethyl ether and thereafter collecting, at 148-150°C, 80.4 g of the intermediate of formula (XIX): 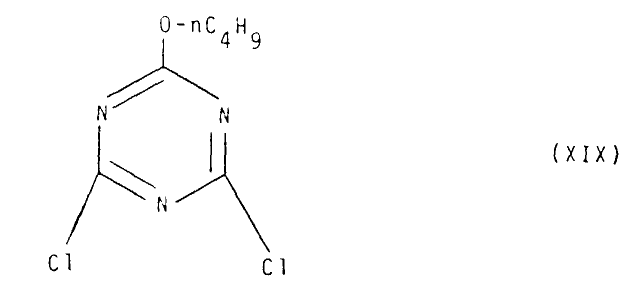 in the form of a colourless liquid having a chlorine content of 31.67% (theory: 31.98%), the structure of which is further confirmed by NMR analysis.

In the above 1 l reactor, this time equipped with a heating bath, 400 ml of ortho-dichlorobenzene, 30 ml of water, 55.5 g of the intermediate (XIX) and, under stirring, 21.5 g of piperazine are introduced.

The temperature of the dispersion rises to 40-45°C.
By working as described in example 3, 55.3 g of the product of formula 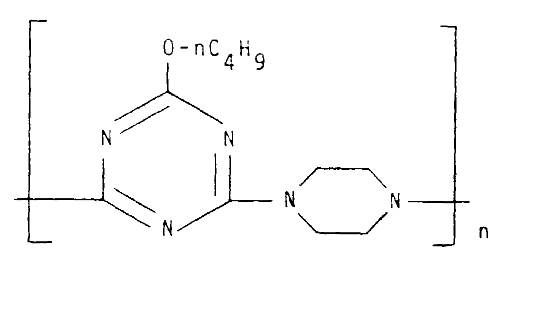 wherein n = 17, are obtained in the form of a white crystalline powder having a m.p. higher than 300°C.

### EXAMPLE 5

In a 2 l reactor equipped like that of example 1, 400 ml of acetone and 100 g of cyanuric acid chloride are introduced.

The suspension is cooled to 0 - 5°C and within 1 hour 23.4 g of piperazine are added.

Always at 0 - 5°C and within 2 hours, 23.3 g of piperazine and 10.8 g of sodium hydroxide, dissolved in 50 ml of water, are simultaneously added in such a manner as to maintain the pH at about 3.

Then the temperature is raised to 20°C and within about two hours 10.8 g of sodium hydroxide dissolved in 50 ml of water are added in such a manner as to maintain the pH at about 5.

The temperature is gradually raised from 20 to 60°C by introducing within about 2 hours, a solution of 21.8 g of sodium hydroxide in 100 ml of water.

Thereafter the whole mass is kept under agitation at 60°C for a further 2 hours and then is cooled to room temperature. The product formed is filtered and washed with water on the filter.

By drying the cake in an oven at 100°C, 104.9 g of the intermediate of formula (XX): 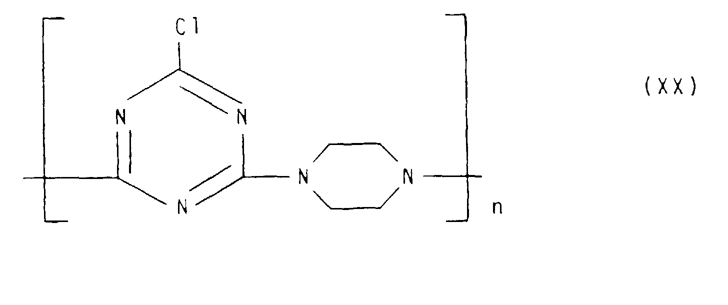 wherein n = 15, are obtained in the form of a white crystalline powder having a m.p. higher than 300°C, and a chlorine content of 17.70% (theory: 17.94%).

The structure of the intermediate (XX) was further confirmed by IR spectroscopic analysis.

In a 1 l reactor equipped with stirrer, thermometer, feeding funnel, reflux condenser and heating bath, 450 ml of water, 98.7 g of the intermediate (XX) and 98.6 g of a 37% by weight hydrochloric acid are introduced.

The mixture is heated to 80°C and is maintained under stiring at this temperature for 2 hours; thereafter it is heated to the boiling point and is kept under reflux for about 6 hours.

Thereafter 60 g of sodium hydroxide dissolved in 150 ml of water are added, whereafter the mixture is allowed to cool to 50°C and the product formed is filtered and washed with water at 50°C on the filter.

By drying the cake in an oven at 100°C, 88.8 g of the product of formula 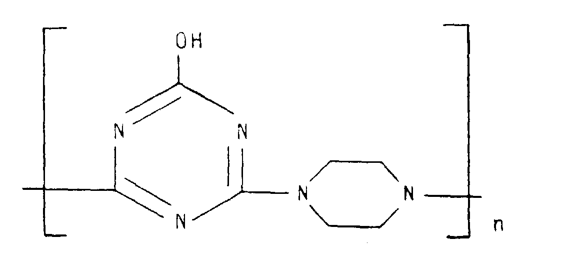 wherein n = 15, are obtained in the form of a white crystalline powder having a m.p. higher than 300°C.

### EXAMPLE 6

In a 2 l reactor equipped like that of example 1, 200 ml of acetonitrile, 200 ml of water and 41.2 g of diethylene triamine are introduced.

The solution is cooled to 0°C and while keeping the temperature in the range of from 0 to 3°C, a solution of 72 g of the intermediate (XVIII) in 500 ml of acetonitrile is added within about 1 hour.

The mixture is then heated to 70°C and at this temperature 84.8 g of sodium carbonate in 250 ml of water are added within 3 hours.

Thereafter the whole mass is heated to boiling and is maintained under reflux for 2 hours.

The distillation of acetonitrile is then started, allowing the dispersion to reach the boiling temperature of water.

The dispersion is kept under reflux for a further 2 hours and thereafter is cooled to room temperature, whereafter the product formed is filtered and washed on the filter with water.

3y drying the cake, 76.6 g of the product of formula 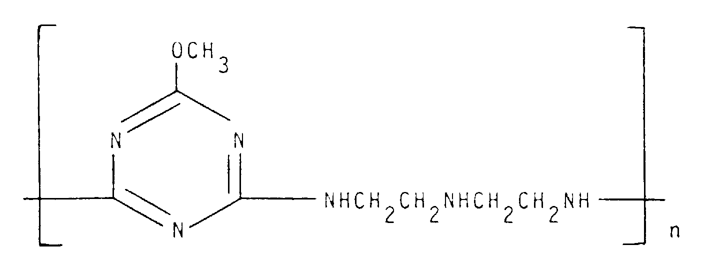 wherein n = 12, are obtained in the form of a white crystalline powder having a m.p. higher than 300°C.

### EXAMPLES 7 - 15

By working under conditions analogous to those described in examples 1 to 6 the products listed in Table 2 are prepared, all of said products having a m.p. higher than 300°C.

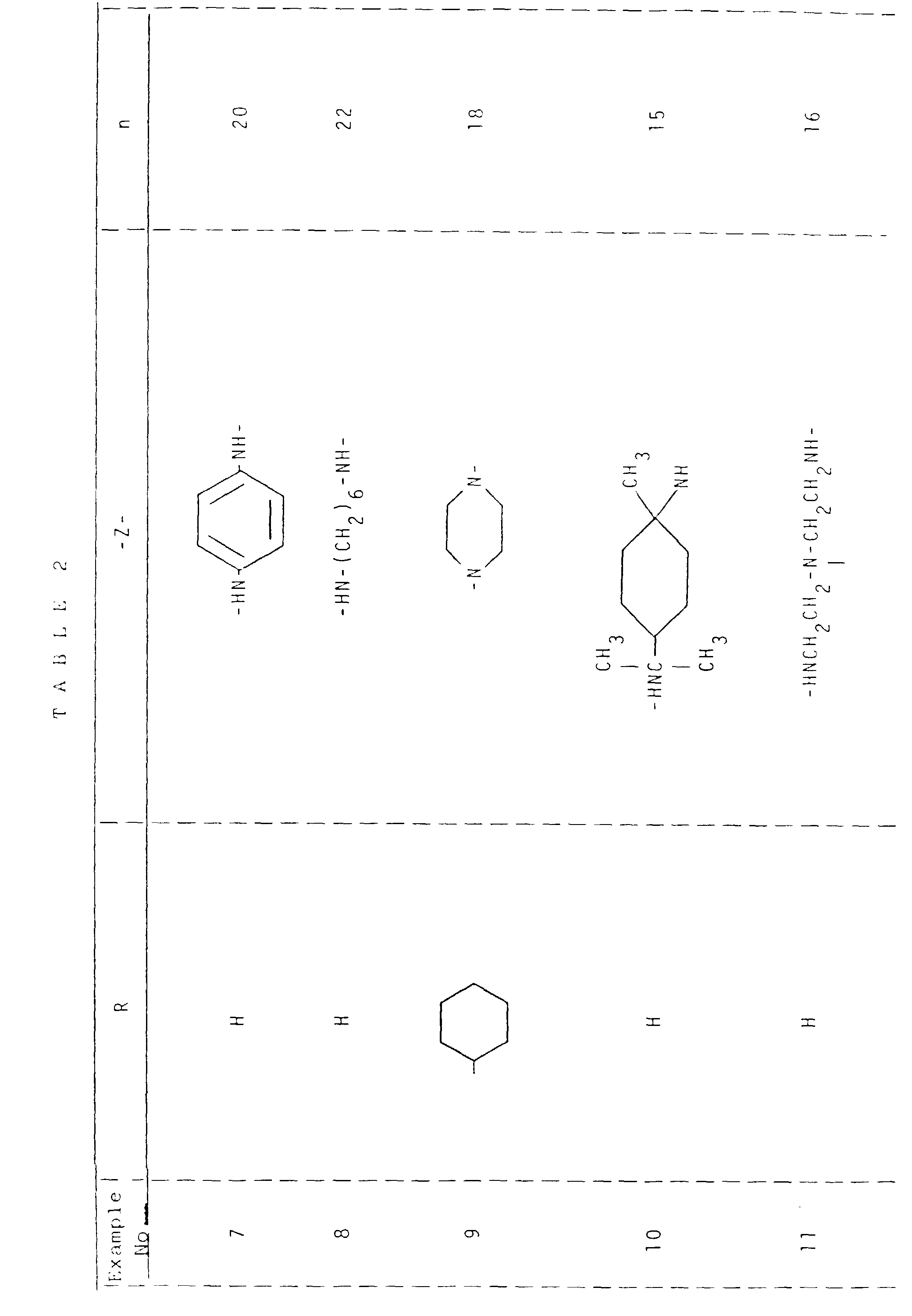

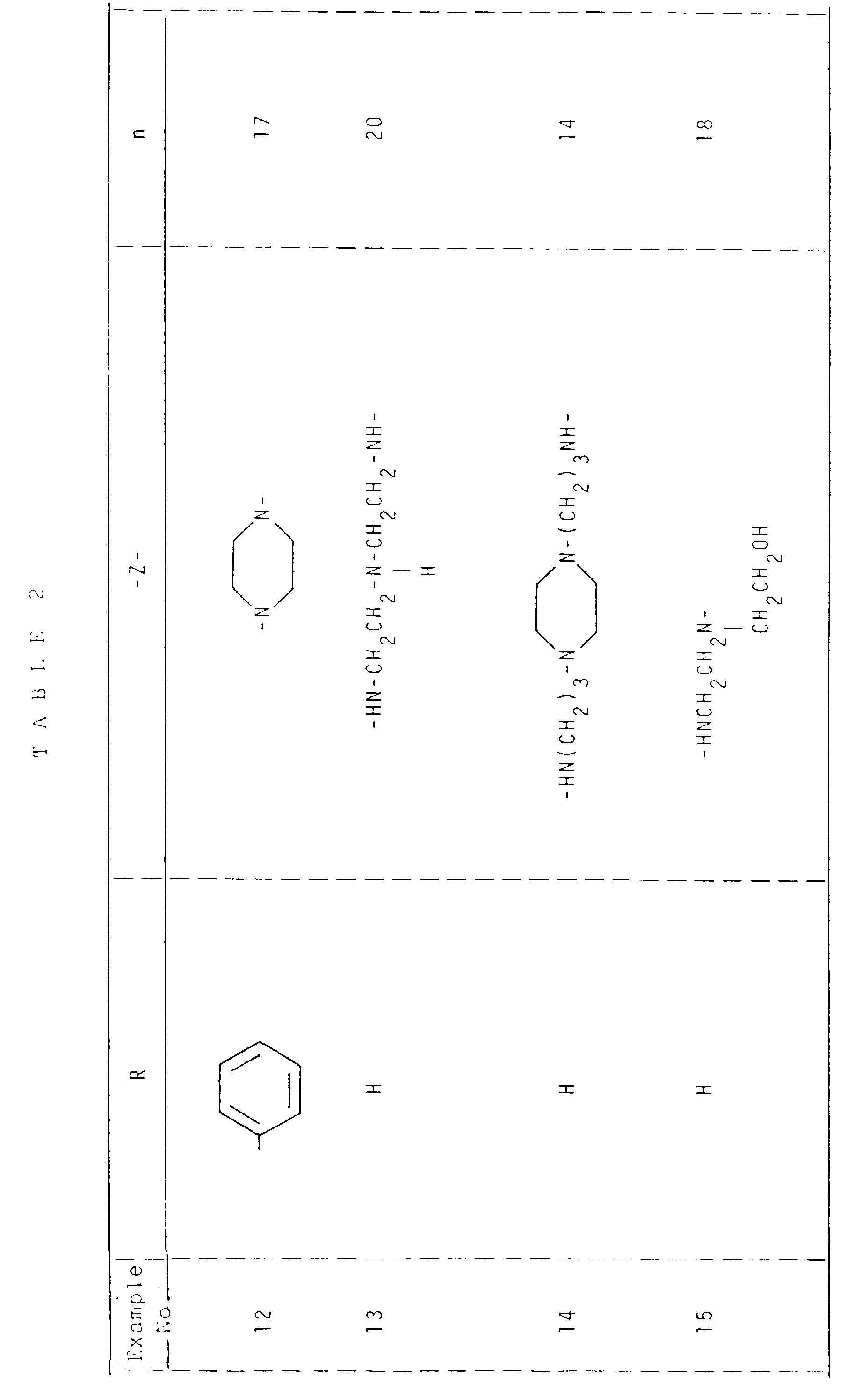

### EXAMPLES 16 TO 47

The tests reported in the following Tables 3 and 4 relate to polymeric compositions containing the products with repeating units of general formula (I) prepared according to the preceding examples.

Specimen having the shape of small slabs of about 3 mm of thickness were prepared by moulding mixtures of granular polymer and additives on a MOORE platen press, with a moulding cycle of 7 minutes and a moulding pressure of about 40 kg/cm².

On the slab specimen thus obtained the level of self-extinguishment was determined by measuring the oxygen index (L.O.I. according to ASTM D-2863-77 in a Stanton Redcroft instrument) and by applying the "Vertical Burning Test" which makes it possible to classify the material according to three rating levels (94 V-O, 94 V-1 and 94 V-2) according to the UL 94 standard (issued by Underwriters Laboratories - U.S.A.).

In Table 3 the values which were obtained by using an isotactic polypropylene in flake form and having a melt flow index of 12 and an insoluble fraction in boiling n-heptane of 96% by weight are reported.

In Table 4 there are reported the values which were obtained by using low-density polyethylene in granular form with a melt flow index of 7; polystyrene granules containing 5% by weight of butadiene rubber and having a melt flow index of 9; thermoplastic polyurethane granules, of polyester grade (ESTANE 54600® by Goodrich) or of polyether grade (ESTANE 58300® by Goodrich), having respective specific gravities of 1.19 and 1.10 g/cm³; an ethylene-propylene elastomeric copolymer containing 45% by weight of propylene; and an acrylonitrile-butadiene-styrene terpolymer having a specific gravity of 1.06 g/cm³ and a melt flow index of 1.6, and containing about 40% of each acrylonitrile and styrene and 20% of butadiene.

**TABLE 3**

| Example No. | Product Ex. No. | PARTS BY WEIGHT | | | | L.O.I. (ASTM D2863) | UL 94 3 mm |
|---|---|---|---|---|---|---|---|
| | | Product | PP(1) | AO(2) | APP(1) | | |
| 16 | 1 | 6.0 | 75 | 1 | 18.0 | 34.5 | V0 |
| 17 | 2 | 6.0 | 75 | 1 | 18.0 | 36.5 | V0 |
| 18 | 3 | 12.0 | 75 | 1 | 12.0 | 31.4 | V0 |
| 19 | 3 | 4.6 | 75 | 1 | 19.4 | 35.5 | V0 |
| 20 | 4 | 6.3 | 74 | 1 | 18.7 | 35.0 | V0 |
| 21 | 5 | 6.0 | 75 | 1 | 18.0 | 34.7 | V0 |
| 22 | 6 | 6.0 | 75 | 1 | 18.0 | 37.0 | V0 |
| 23 | 7 | 6.8 | 72 | 1 | 20.2 | 31.8 | V0 |
| 24 | 8 | 6.0 | 72 | 1 | 21.0 | 32.4 | V0 |
| 25 | 9 | 6.4 | 73 | 1 | 19.6 | 33.2 | V1 |
| 26 | 10 | 7.0 | 71 | 1 | 21.0 | 32.9 | V0 |
| 27 | 11 | 6.0 | 75 | 1 | 18.0 | 35.9 | V0 |
| 28 | 12 | 6.2 | 74 | 1 | 18.8 | 30.8 | V1 |
| 29 | 13 | 6.0 | 75 | 1 | 18.0 | 36.5 | V0 |
| 30 | 14 | 6.8 | 75 | 1 | 17.2 | 33.2 | V0 |
| 31 | 15 | 6.8 | 75 | 1 | 17.2 | 35.8 | V0 |
| | | | | | | | |
| 32 | 3 | 6.4 | 76 | 1 | 16.6^{(*)} | 34.0 | V0 |
| | | | | | | | |
| 33 | 13 | 6.8 | 75 | 1 | 17.2^{(*)} | 36.3 | V0 |
| | | | | | | | |
| 34 | 3 | 6.2 | 73 | 1 | 19.8⁽³⁾ | 32.8 | V0 |
| | | | | | | | |
| 35 | 6 | 7.8 | 72 | 1 | 19.2⁽⁴⁾ | 34.4 | V0 |

| | | | | | | | |
|---|---|---|---|---|---|---|---|
| (1) PP = polypropylene APP = Ammonium polyphosphate- Exolit ®422 (Hoechst) | | | | | | | |
| * APP = ammonium polyphosphate microincapsulated with melamine-formaldehyde resin Exolit® 462 (Hoechst) | | | | | | | |
| (2) AO = antioxidant Mixture of 2 parts of dilauryl thiopropionate and 1 part of pentaerythritol tetra[3-(3,5-di-tert-butyl-4-hydroxyphenyl)propionate] | | | | | | | |
| (3) monoammonium salt of 1-aminoethane-1,1-diphosphonic acid | | | | | | | |
| (4) monoammonium salt of 1-hydroxyethane-1,1-diphosphonic acid | | | | | | | |

**TABLE 4**

| Example No. | Polymeric supp. (2) | Product EX. No. | PARTS BY WEIGHT | | | | L.O.I. (ASTM-D2863) | UL 94 3 mm |
|---|---|---|---|---|---|---|---|---|
| | | | Product | Polymer | A0 (3) | APP(1) | | |
| 36 | LDPE | 1 | 9.7 | 65 | 1 | 24.3 | 32.5 | V0 |
| 37 | | 3 | 7.2 | 70 | 1 | 21.8 | 31.5 | V0 |
| 38 | | 5 | 7.3 | 70 | 1 | 21.7 | 35.2 | V0 |
| 39 | | 13 | 8.5 | 65 | 1 | 25.5 | 33.8 | V0 |
| 40 | HIPS | 5 | 9.7 | 60 | 1 | 29.3 | 33.6 | V0 |
| 41 | PU estere | 2 | 7.2 | 70 | 1 | 21.8 | 36.8 | V0 |
| 42 | | 5 | 7.2 | 70 | 1 | 21.8 | 39.0 | V0 |
| 43 | | 6 | 7.2 | 70 | 1 | 21.8 | 38.0 | V0 |
| 44 | PU ether | 2 | 6.8 | 70 | 1 | 22.2 | 34.3 | V0 |
| 45 | PP/PE | 3 | 8.3 | 70 | 1 | 20.7 | 35.0 | V0 |
| 46 | | 6 | 7.3 | 70 | 1 | 21.7 | 33.8 | V0 |
| 47 | ABS | 15 | 9.2 | 60 | 1 | 29.8 | 33.2 | V0 |

| | | | | | | | | |
|---|---|---|---|---|---|---|---|---|
| (1) APP = Ammonium polyphosphate- Exolit® 422 (Hoechst) | | | | | | | | |
| (2) LPDE = low density polyethylene HIPS = polystyrene containing 5% of butadiene rubber PU (ester) = polyurethane polyester PU (ether) = polyurethane polyether PP/PE = propylene-ethylene copolymer ABS = acrylonitrile-butadiene-styrene terpolymer | | | | | | | | |
| (3) AO = antioxidant Mixture of 2 parts of dilauryl thiopropionate and 1 part of pentaerythritol tetra[3-(3,5-di-tert-butyl-4-hydroxyphenyl) propionate] | | | | | | | | |

## Claims

1. Self-extinguishing polymeric compositions comprising:
a) from 89 to 40 parts by weight of thermoplastic polymer and/or of polymer having elastomeric properties;
b) from 8 to 33 parts by weight of one or more ammonium and/or amine phosphates and/or phosphonates;
c) from 3 to 27 parts by weight of one or more oligomeric 2,4-diamino-6-hydroxy-1,3,5-triazine derivatives having 2 to 50 repeating units of general formula (I): 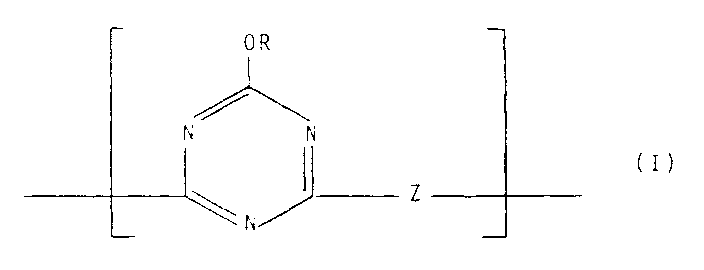
wherein:
R = is hydrogen; (C₂-C₆)-alkenyl; (C₆-C₁₂)-cycloalkyl; (C₆-C₁₂) -alkylcycloalkyl; (C₆-C₁₂)-aryl; (C₇-C₁₂)-aralkyl; or ⁅CₘH₂ₘ⁆Y,
m being an integer of from 1 to 8, and
Y being selected from hydrogen; CN; -O(C₁-C₄)-alkyl; -O(C₂-C₄)-alkenyl; (C₆-C₁₂)-cycloalkyl; (C₆-C₁₂)-alkylcycloalkyl; -O(C₆-C₁₂)-aryl; -N(R₁)₂, wherein the groups R₁, equal or different from each other, are (C₁-C₄)-alkyl or (C₃-C₄)-alkenyl; and an N-heterocyclic radical linked to the alkyl chain through the nitrogen atom and optionally containing another heteroatom; and
Z is a divalent or polyvalent radical selected from those of the following formulae: 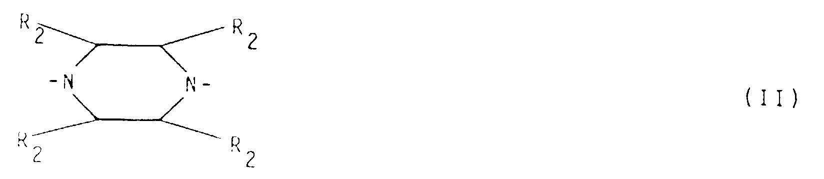
wherein the groups R₂, the same or different from each other, represent hydrogen and (C₁-C₄)-alkyl; 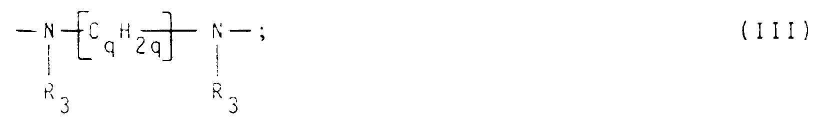 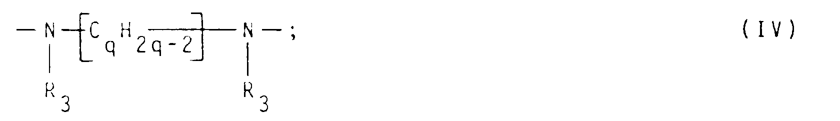 wherein q is an integer of from 2 to 14 and R₃ is hydrogen; (C₁-C₄)-alkyl; (C₂-C₆)-alkenyl; or (C₁-C₄)-hydroxyalkyl; 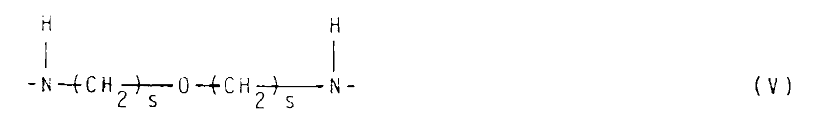 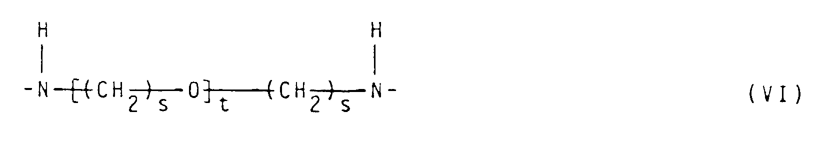 wherein s is an integer of from 2 to 5 and t is an integer of from 1 to 3; 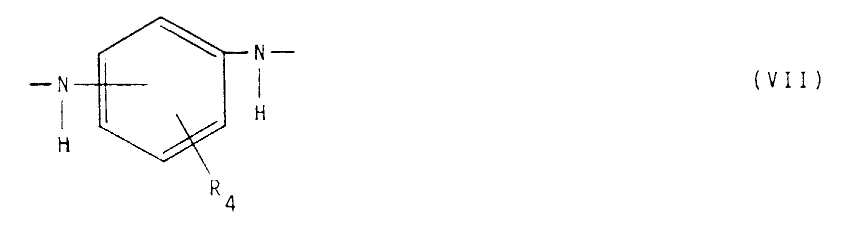 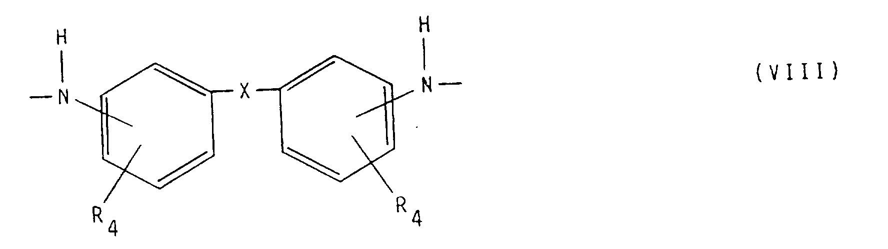 wherein:
X is a direct carbon-carbon bond; O; S; S-S; SO; SO₂; NH; NHSO₂, NHCO; N=N; or CH₂; and
R₄ is hydrogen; hydroxy; (C₁-C₄)-alkyl; or (C₁-C₄)-alkoxy; 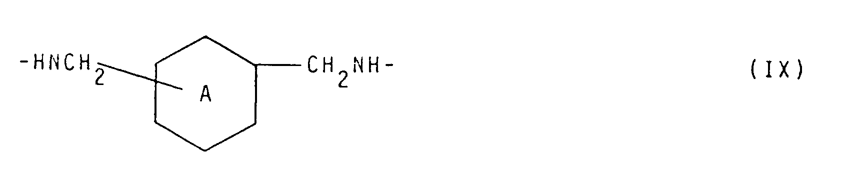
wherein A is a saturated or unsaturated ring; 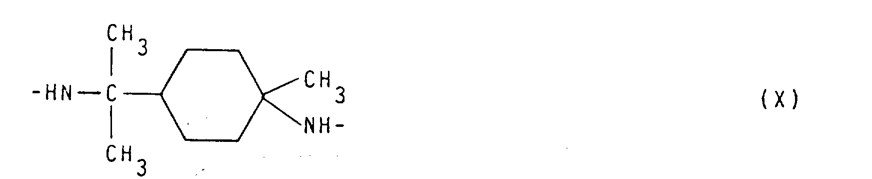 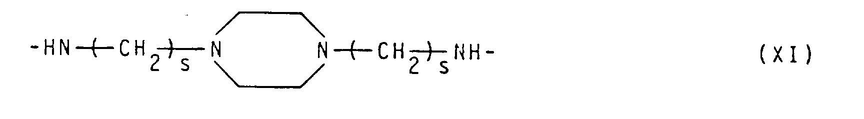 wherein s is an integer of from 2 to 5; 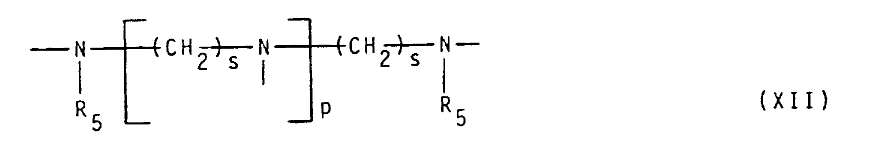 wherein R₅ is hydrogen or (C₁-C₄)-alkyl; p is an integer of from 1 to 5; and the subscripts s, the same or different from each other, are integers of from 2 to 5; 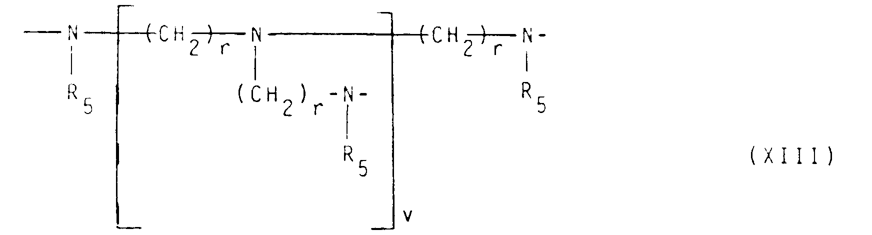 wherein:
R₅ is hydrogen or (C₁-C₄)-alkyl;
r is an integer of from 2 to 4; and
v is 1 or 2.

2. Compositions according to claim 1, wherein R in general formula (I) is hydrogen or ⁅CₘH₂ₘ⁆Y wherein m is an integer of from 1 to 4 and Y is hydrogen.

3. Compositions according to claim 1, wherein Y is an N-heterocyclic radical selected from pyrrolidyl; piperidyl; morpholyl; thiomorpholyl; piperazyl; 4-methylpiperazyl; and 4-ethylpiperazyl.

4. Compositions according to any one of the preceding claims, wherein the ammonium phosphates (b) have the general formula (NH₄)ₙ₊₂PₙO₃ₙ₊₁ wherein n is an integer of at least 2 or have the general formula (NH₄PO₃)ₙ wherein n ranges from 50 to 500.

5. Compositions according to any one of claims 1 to 4 wherein the amine phosphates (b) are selected from dimethylammonium phosphate; diethylammonium phosphate; ethylenediamine phosphate; melamine ortho-phosphate; and melamine pyrophosphate.

6. Compositions according to any one of claims 1 to 5, wherein the ammonium phosphonates (b) are the mono- or polysubstituted ones and are selected from salts of mono- and polyphosphonic acids.

7. Compositions according to any one of claims 1 to 6, wherein the polymer (a) is selected from acrylonitrile/ butadiene/styrene terpolymers (ABS) ; styrene/acrylonitrile (SAN) copolymers; polyurethanes; poly(ethyleneterephthalate) ; poly(butyleneterephthalate); polyamides; and polymers and copolymers of olefins of general formula R'-CH=CH₂ wherein R' is hydrogen, (C₁-C₈)-alkyl or (C₆-C₁₀)-aryl, particularly
(1) HDPE, LLDPE and LDPE polyethylene;
(2) isotactic or prevailingly isotactic polypropylene;
(3) crystalline copolymers of propylene and minor amounts of ethylene and/or other alpha-olefins, such as butene-1, hexene-1, octene-1 and 4-methylpentene-1;
(4) heterophasic compositions comprising (A) a fraction of ethylene or propylene homopolymer or of copolymers as specified under (3) and (B) a copolymeric fraction composed of elastomeric copolymers of ethylene and an alpha-olefin, optionally containing minor amounts of diene, wherein the alpha-olefin is preferably selected from propylene and butene-1;
(5) elastomeric copolymers of ethylene and alpha-olefins optionally containing minor amounts of diene.

8. Molded articles, obtained from the compositions of any one of the preceding claims.

## Patentansprüche

1. Selbstverlöschende polymere Zusammensetzungen, umfassend:
a) 89 bis 40 Gewichtsteile thermoplastisches Polymer und/oder Polymer mit elastomeren Eigenschaften;
b) 8 bis 33 Gewichtsteile eines oder mehrerer Ammonium- und/oder Aminphosphate und/oder -phosphonate;
c) 3 bis 27 Gewichtsteile eines oder mehrerer oligomerer 2,4-Diamino-6-hydroxy-1,3,5-triazin-Derivate mit 2 bis 50 wiederkehrenden Einheiten der allgemeinen Formel (I): 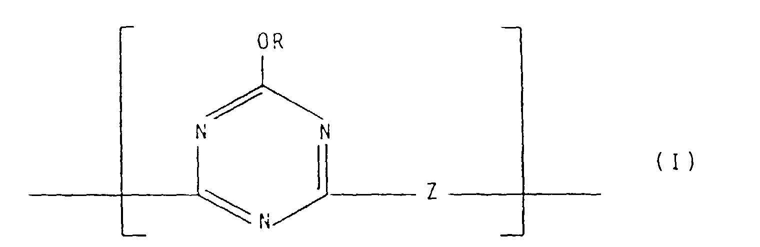
worin:
R = Wasserstoff; (C₂-C₆)-Alkenyl; (C₆-C₁₂)-Cydoalkyl; (C₆-C₁₂)-Alkylcycloalkyl; (C₆-C₁₂)-Aryl; (C₇-C₁₂)-Aralkyl; oder ⁅CₘH₂ₘ⁆Y,
wobei m eine ganze Zahl von 1 bis 8 ist und
Y ausgewählt ist aus Wasserstoff; CN, -O(C₁-C₄)-Alkyl; -O(C₂-C₄)-Alkenyl; (C₆-C₁₂)-Cycloalkyl; (C₆-C₁₂)-Alkylcycloalkyl; -O(C₆-C₁₂)-Aryl; -N(R₁)₂, worin die Gruppen R₁, gleich oder verschieden voneinander, (C₁-C₄)-Alkyl oder (C₃-C₄)-Alkenyl bedeuten; und einem N-heterocyclischen Rest, der über das Stickstoffatom an die Alkylkette gebunden ist und gegebenenfalls ein weiteres Heteroatom enthält; und
Z ein zweiwertiger oder mehrwertiger Rest ist, der ausgewählt ist aus denjenigen mit den folgenden Formeln: 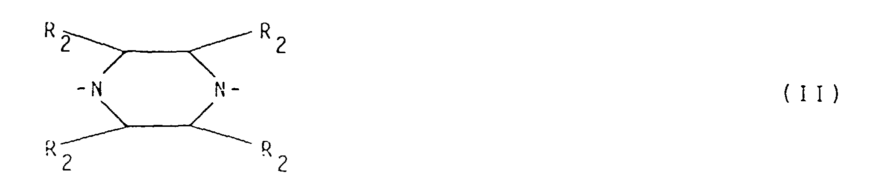
worin die Gruppen R₂, gleich oder verschieden voneinander, Wasserstoff und (C₁-C₄)-Alkyl darstellen; 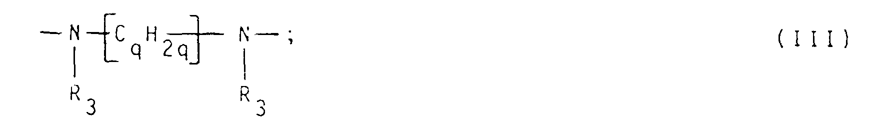 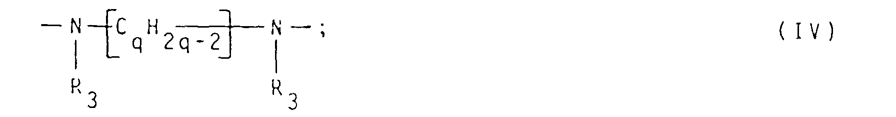 worin q eine ganze Zahl von 2 bis 14 ist und R₃ Wasserstoff; (C₁-C₄)-Alkyl; (C₂-C₆)-Alkenyl; oder (C₁-C₄)-Hydroxyalkyl bedeutet; 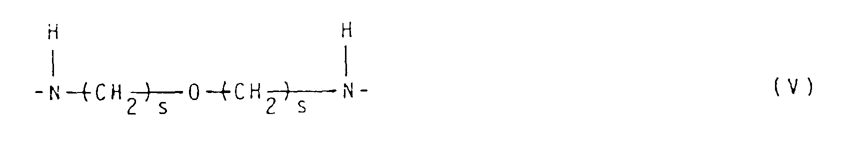 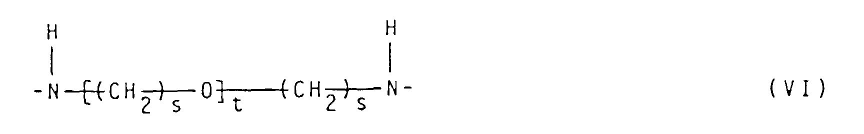 worin s eine ganze Zahl von 2 bis 5 ist und t eine ganze Zahl von 1 bis 3 ist; 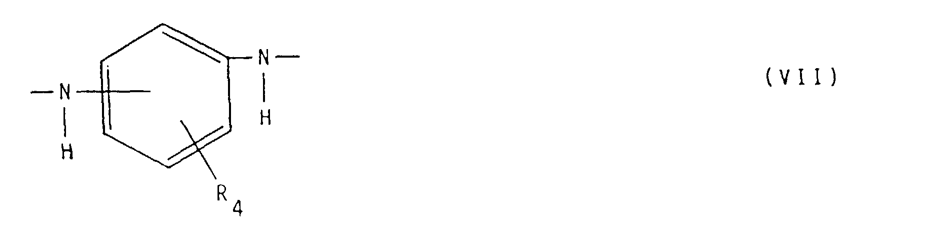 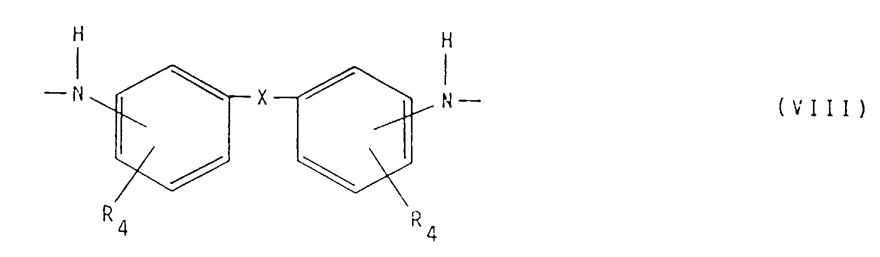 worin:
X für eine direkte Kohlenstoff-Kohlenstoff-Bindung; O; S; S-S; SO; SO₂; NH; NHSO₂; NHCO; N=N; oder CH₂ steht; und
R₄ Wasserstoff; Hydroxy; (C₁-C₄)-Alkyl; oder (C₁-C₄)-Alkoxy repräsentiert; 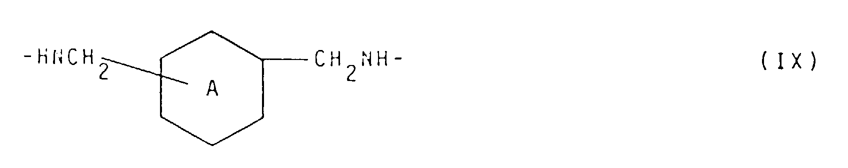
worin A ein gesättigter oder ungesättigter Ring ist; 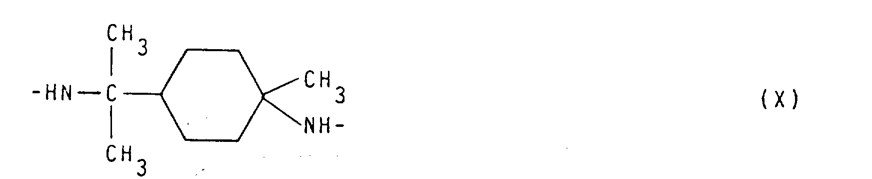 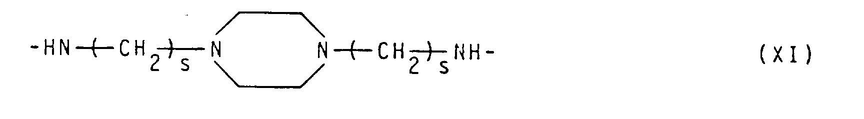 worin s eine ganze Zahl von 2 bis 5 ist; 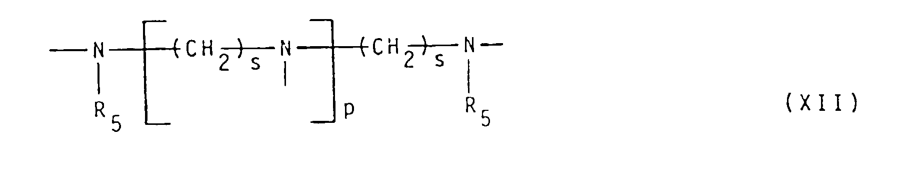 worin R₅ Wasserstoff oder (C₁-C₄)-Alkyl bedeutet; p eine ganze Zahl von 1 bis 5 ist; und die Indices s, gleich oder verschieden voneinander, ganze Zahlen von 2 bis 5 sind; 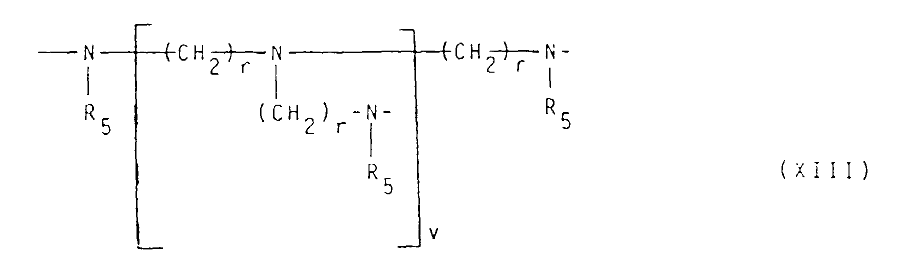 worin:
R₅ Wasserstoff oder (C₁-C₄)-Alkyl bedeutet;
r eine ganze Zahl von 2 bis 4 ist; und
v 1 oder 2 darstellt.

2. Zusammensetzungen nach Anspruch 1, in denen R in der allgemeinen Formel (I) Wasserstoff oder ⁅CₘH₂ₘ⁆Y bedeutet, worin m eine ganze Zahl von 1 bis 4 ist und Y Wasserstoff darstellt.

3. Zusammensetzungen nach Anspruch 1, in denen Y ein aus Pyrrolidyl; Piperidyl; Morpholyl; Thiomorpholyl; Piperazyl; 4-Methylpiperazyl; und 4-Ethylpiperazyl ausgewählter heterocyclischer Rest ist.

4. Zusammensetzungen nach irgendeinem der vorangehenden Ansprüche, in denen die Ammoniumphosphate (b) die allgemeine Formel (NH₄)ₙ₊₂PₙO₃ₙ₊₁ aufweisen, worin n eine ganze Zahl von mindestens 2 ist, oder die allgemeine Formel (NH₄PO₃)ₙ aufweisen, worin n im Bereich von 50 bis 500 liegt.

5. Zusammensetzungen nach irgendeinem der Ansprüche 1 bis 4, in denen die Aminphosphate (b) aus Dimethylammoniumphosphat; Diethylammoniumphosphat; Ethylendiaminphosphat; Melaminorthophosphat; und Melaminpyrophosphat ausgewählt sind.

6. Zusammensetzungen nach irgendeinem der Ansprüche 1 bis 5, in denen die Ammoniumphosphonate (b) die mono- oder polysubstituierten sind und aus Salzen von Mono- und Polyphosphonsäuren ausgewählt sind.

7. Zusammensetzungen nach irgendeinem der Ansprüche 1 bis 6, in denen das Polymer (a) ausgewählt ist aus Acrylnitril/Butadien/Styrol-Terpolymeren (ABS); Stryol/Acrylnitril (SAN)-Copolymeren; Polyurethanen; Poly(ethylenterephthalat); Poly(butylenterephthalat); Polyamiden; und Polymeren und Copolymeren von Olefinen der allgemeinen Formel R'-CH=CH₂, worin R' für Wasserstoff, (C₁-C₈)-Alkyl oder (C₆-C₁₀)-Aryl steht, insbesondere
(1) HDPE-, LLDPE- und LDPE-Polyethylen;
(2) isotaktischem oder überwiegend isotaktischem Polypropylen;
(3) kristallinen Copolymeren von Propylen und kleineren Mengen an Ethylen und/oder anderen alpha-Olefinen, wie beispielsweise Buten-1, Hexen-1, Octen-1 und 4-Methylpenten-1;
(4) heterophasischen Zusammensetzungen, umfassend (A) eine Fraktion von Ethylen- oder Propylen-Homopolymer oder von Copolymeren wie unter (3) angegeben und (B) eine copolymere Fraktion, die aus elastomeren Copolymeren von Ethylen und einem alpha-Olefin, gegebenenfalls kleinere Mengen an Dien enthaltend, zusammengesetzt ist, wobei das alpha-Olefin vorzugsweise aus Propylen und Buten-1 ausgewählt ist;
(5) elastomeren Copolymeren von Ethylen und alpha-Olefinen, die gegebenenfalls kleinere Mengen an Dien enthalten.

8. Formkörper, erhalten aus den Zusammensetzungen nach irgendeinem der vorangehenden Ansprüche.

## Revendications

1. Compositions polymères ignifuges comprenant :
a) de 89 à 40 parties en poids de polymère thermoplastique et/ou d'un polymère ayant des propriétés élastomères,
b) de 8 à 33 parties en poids d'un ou de plusieurs composés de type phosphate et/ou phosphonate d'ammonium et/ou d'amine;
c) de 3 à 27 parties en poids d'un ou de plusieurs dérivés d'oligomères de 2,4-diamino-6-hydroxy-1,3,5-triazine comportant de 2 à 50 motifs répétitifs de formule générale 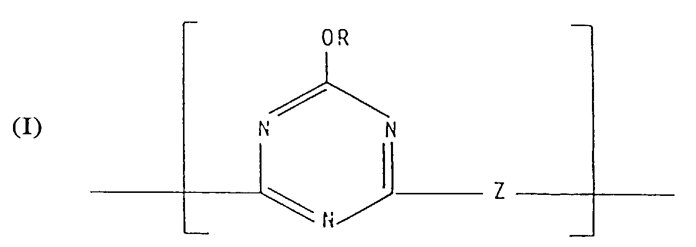
dans laquelle
R représente un atome d'hydrogène, un résidu alcényle en C₂₋₆, cycloalkyle en C₆₋₁₂, alkylcycloalkyle en C₆₋₁₂, aryle en C₆₋₁₂, aralkyle en C₇₋₁₂ ou -[CₘH₂ₘ]-Y, où m est un nombre entier compris entre 1 et 8 et Y est choisi parmi un atome d'hydrogène, un résidu C=N, -O-(alkyle en C₁₋₄), -O-(alcényle en C₂₋₄), cycloalkyle en C₆₋₁₂, alkylcycloalkyle en C₆₋₁₂, -O-(aryle en C₆₋₁₂), -N(R₁)₂ où les groupes R₁, identiques ou différents l'un de l'autre, représentent des groupes alkyle en C₁₋₄ ou alcényle en C₃₋₄, et un radical hétérocyclique azoté lié à la chaîne alkyle par l'intermédiaire de l'atome d'azote et contenant éventuellement un autre hétéroatome; et
Z est un radical divalent ou polyvalent choisi parmi ceux d'une des formules suivantes 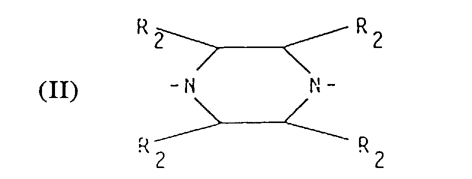
dans laquelle les groupes R₂, identiques ou différents l'un de l'autre, représentent des atomes d'hydrogène ou des groupes alkyle en C₁₋₄; 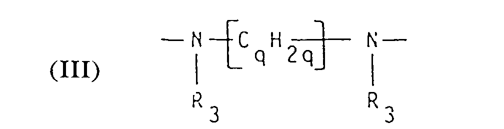 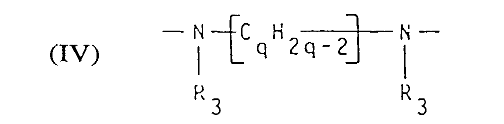 dans lesquelles q représente un nombre entier compris entre 2 et 14 et R₃ est un atome d'hydrogène, un groupe alkyle en C₁₋₄, alcényle en C₂₋₆ ou hydroxyalkyle en C₁₋₄, 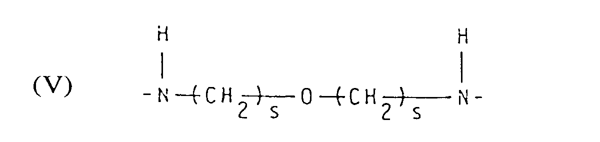 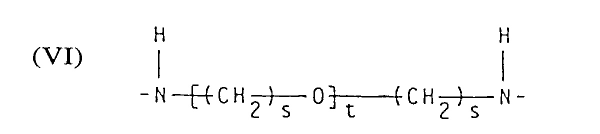 où s est un nombre entier compris entre 2 et 5 et t est un nombre entier compris entre 1 et 3; 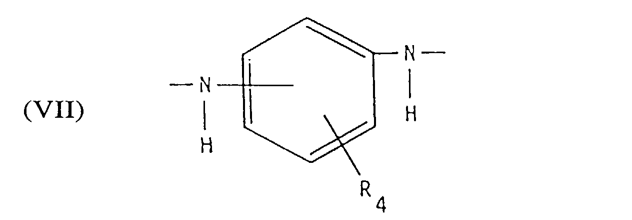 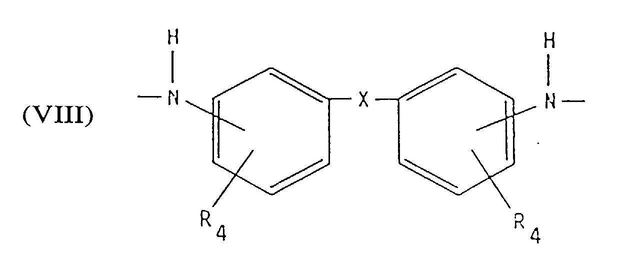 dans lesquelles X est une liaison directe carbone-carbone, un atome d'oxygène ou de soufre, un groupe S-S, S-O, SO₂, NH, NHSO₂, NHCO, N=N ou CH₂, et R₄ est un atome d'hydrogène, un groupe hydroxy, alkyle en C₁₋₄ ou alkyloxy en C₁₋₄, 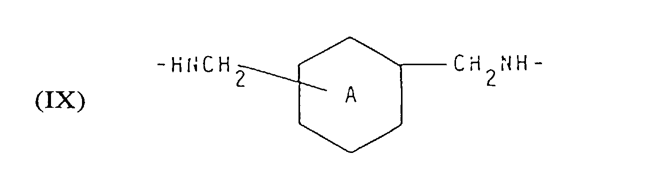 où A est un cycle saturé ou insaturée, 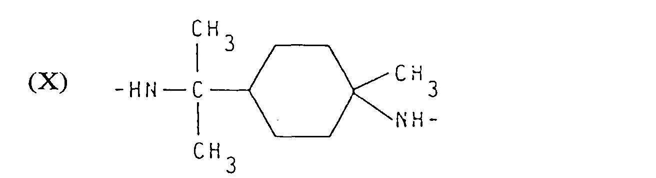 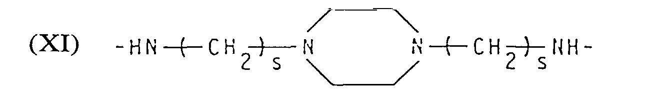 où s est un nombre entier compris entre 2 et 5; 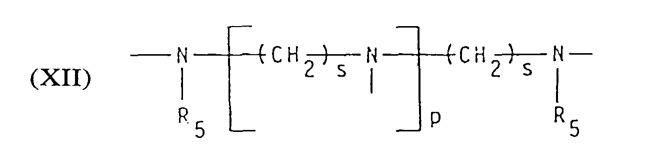 où R₅ est un atome d'hydrogène ou un groupe alkyle en C₁₋₄, p est un nombre entier compris entre 1 et 5 et les indices s, identiques ou différents l'un de l'autre, sont des nombres entiers compris entre 2 et 5, 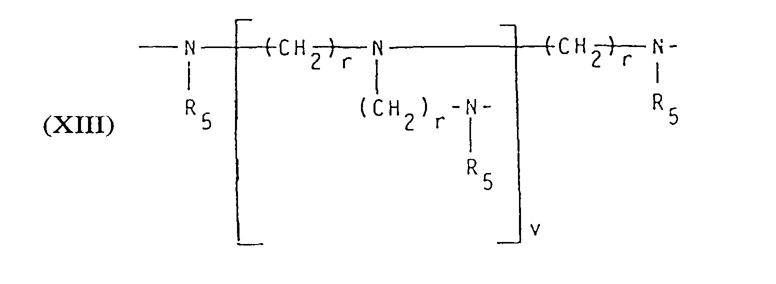 où R₅ est un atome d'hydrogène ou un groupe alkyle en C₁₋₄, r est un nombre entier compris entre 2 et 4 et v vaut 1 ou 2.

2. Compositions conformes à la revendication 1 dans lesquelles R dans la formule générale (I) est un atome d'hydrogène ou un groupe -[CₘH₂ₘ]-Y où m est un nombre entier compris entre 1 et 4 et Y est un atome d'hydrogène.

3. Compositions conformes à la revendication 1 dans lesquelles Y est un radical hétérocyclique azoté choisi parmi les résidus pyrrolidyle, pipéridyle, morpholyle, thiomorpholyle, pipérazyle, 4-méthylpipérazyle et 4-éthylpipérazyle.

4. Compositions conformes à l'une quelconque des revendications précédentes dans lesquelles les phosphates d'ammonium (b) correspondent à la formule générale (NH₄)ₙ₊₂PₙO₃ₙ₊₁ dans laquelle n est un nombre entier au moins égal à 2 ou correspondent à la formule générale (NH₄PO₃)ₙ où n va de 50 à 500.

5. Composition conformes à l'une quelconque des revendications 1 à 4 dans lesquelles les phosphates d'amine (b) sont choisis parmi le phosphate de diméthylammonium, le phosphate de diéthylammonium, le phosphate d'éthylènediamine, l'orthophosphate de mélamine et le pyrophosphate de mélamine.

6. Compositions conformes à l'une quelconque des revendications 1 à 5, dans lesquelles les phosphonates d'ammonium (b) sont mono- ou polysubstitués et sont choisis parmi les sels d'acides mono- et polyphosphoniques.

7. Compositions conformes à l'une quelconque des revendications 1 à 6, dans lesquelles le polymère (a) est choisi parmi les terpolymères acrylonitrile/butadiène/styrène, les copolymères styrène/acrylonitrile (SAN), les polyuréthannes, le poly(éthylène téréphtalate), le poly(butylène téréphtalate), les polyamides et des polymères et copolymères d'oléfines de formule générale R'-CH=CH₂ où R' est un atome d'hydrogène, un groupe alkyle en C₁₋₈ ou aryle en C₆₋₁₀, en particulier
(1) du polyéthylène de type PE-HD, PE-BDL ou PE-BD,
(2) du polypropylène isotactique ou majoritairement isotactique;
(3) des copolymères cristallins de propylène et de faibles quantités d'éthylène et/ou d'autre α-oléfines telles que le butène-1, l'hexène-1, l'octène-1 et le 4-méthylpentène-1;
(4) des compositions hétérophasiques comprenant
(A) une fraction d'un homopolymère d'éthylène ou de propylène ou d'un des copolymères spécifiés sous (3) et
(B) une fraction copolymère composée de copolymères élastomères d'éthylène et d'une α-oléfine, contenant éventuellement de faibles quantités de diène, où l'α-oléfine est de préférence choisie parmi le propylène et le butène-1;
(5) des copolymères élastomères d'éthylène et d'α-oléfines pouvant contenir de faibles quantités de diène.

8. Articles moulés obtenus à partir des compositions conformes à l'une quelconque des revendications précédentes.
